**Europäisches Patentamt**

**European Patent Office**

**Office européen des brevets**

(19)

(11) Veröffentlichungsnummer: **0 199 034**

**A1**

(12)

# EUROPÄISCHE PATENTANMELDUNG

(21) Anmeldenummer: **86103010.4**

(22) Anmeldetag: **06.03.86**

(51) Int. Cl.⁴: **A 61 K 9/48**
**A 61 K 9/66**

(30) Priorität: **25.04.85 DE 3546452**

(43) Veröffentlichungstag der Anmeldung:
**29.10.86 Patentblatt 86/44**

(84) Benannte Vertragsstaaten:
**AT BE CH DE FR GB IT LI LU NL SE**

(71) Anmelder: **R.P. Scherer GmbH**
**Gammelsbacher Strasse 2 Postfach 1243**
**D-6930 Eberbach/Baden(DE)**

(71) Anmelder: **Theurer, Karl Eugen, Prof.Dr.med.**
**Brunnwiesenstrasse 23**
**D-7302 Ostfildern 1(DE)**

(72) Erfinder: **Theurer, Karl E., Prof. Dr.**
**Brunnwiesenstrasse 23**
**D-7302 Ostfildern 1 (Ruit)(DE)**

(72) Erfinder: **Fischer, Gerhard, Dr.**
**Hohenstaufenstrasse 28/1**
**D-6930 Eberbach/Baden(DE)**

(74) Vertreter: **Werner, Hans-Karsten, Dr. et al,**
**Deichmannhaus am Hauptbahnhof**
**D-5000 Köln 1(DE)**

(54) Weichgelatinekapseln und Verfahren zu ihrer Herstellung.

(57) Weichgelatinekapseln bestehend aus einer Kapselhülle und einer Kapselfüllung, die gegebenenfalls Wirkstoffe und/oder Diätmittel enthält, insbesondere Zell- und Organbestandteile nach DE-OS 33 02 319, haben eine Kapselfüllung bestehend aus einem Gemisch aus 2 bis 20 Gew.-% Glycerin und 80 bis 98 Gew.-% Honig, Invertzukker oder Zuckersirupen, in denen gegebenenfalls die Wirkstoffe und/oder Diätmittel gelöst oder dispergiert sind. Gewünschtenfalls können die Weichgelatinekapseln noch gas- und wasserdampfdicht eingesiegelt werden.

EP 0 199 034 A1

Croydon Printing Company Ltd.

Gegenstand der vorliegenden Erfindung sind Weichgelatinekapseln bestehend aus einer Kapselhülle und einer Kapselfüllung, die gegebenenfalls Wirkstoffe und/oder Diätmittel enthält, sowie Verfahren zu ihrer Herstellung.

Wirkstoffe und Diätmittel, insbesondere solche mit Zell- und Organbestandteilen (korpuskulären Extrakten, Eiweißstoffen, Nukleinsäuren und deren Bestandteilen) gemäß DE-OS 33 02 319, welche Honig, Invertzucker oder andere Zuckerstoffe enthalten, konnten bislang nicht in Weichgelatinekapseln, insbesondere Kau- oder Zerbeißkapseln, abgefüllt werden. Die Ursachen hierfür sind z.B. in Lachman, Theory and practice of industrial pharmacy, Lea and Febiger, Philadelphia, 2. Ausgabe, beschrieben. Nach Lachman sind gleich mehrere Gründe für die vorhandenen Probleme verantwortlich. Zum einen können Honig oder Zuckerlösungen produktionstechnisch bedingt nur bis max. 40 °C abgefüllt werden, was einerseits eine große Klebrigkeit und andererseits eine relativ hohe Viskosität des Füllgutes zur Folge hat. Zum anderen sind wasserhaltige Lösungen normalerweise nur bis zu einem Wassergehalt von 10 % verkapselbar, weil sonst das Wasser die Kapselhülle angreift.

Die Erfindung hat sich die Aufgabe gestellt, Weichgelatinekapseln bestehend aus einer Kapselhülle und einer Kapselfüllung, die gegebenenfalls Wirkstoffe und/oder Diätmittel enthält, zu entwickeln, bei denen die Kapselfüllung Honig, Invertzucker oder Zuckersirupe enthält, in denen gegebenenfalls die Wirkstoffe oder Diätmittel gelöst oder dispergiert sind.

Es wurde gefunden, daß tatsächlich stabile und nicht austrocknende Kapseln herstellbar sind, wenn man dem Honig, Invertzucker oder Zuckersirup 2 bis 20 Gew.-% Glycerin beimischt. Dieser Zusatz von Glycerin verhindert einerseits, daß die Kapseln von den wässrigen Zuckerlösungen angegriffen werden und zum anderen, daß die wässrigen Zuckerlösungen durch die Gelatinehülle hindurch zu viel ihres Wassergehaltes verlieren. Weiterhin bewirkt der Zusatz von Glycerin, daß die Viskosität und Klebrigkeit der Kapselfüllung herabgesetzt werden.

Bei einem Glyceringehalt von unter 2 % ist die Wirkung noch nicht ausreichend. Mehr als 20 % Glycerin ist unerwünscht, da dies zum Ausfällen des Zuckers, Invertzuckers oder Zuckersirups führen kann. Vorzugsweise werden daher Glycerinmengen von 10 bis 15 % eingesetzt. Es ist erstaunlich, daß der relativ hohe Glyceringehalt im Honig, Invertzucker oder Zuckersirup zu keinen Störungen bei der Verkapselung führt, da in der Kapsel bei der Herstellung zwar gewisse Mengen Glycerin als Weichmacher vorhanden sein sollen, diese jedoch nicht zu hoch sein dürfen. Anscheinend werden die erfindungsgemäß zugesetzten Mengen Glycerin in erheblichem Umfang durch die wässrigen Zuckerlösungen gebunden, so daß es zu keinen Störungen bei der Verkapselung kommt. Vorzugsweise kann man die Rezeptur für die Kapselhülle so wählen, daß sie eine verminderte Menge Glycerin als Weichmacher enthält. Beim Trocknen der abgefüllten Kapseln wandert dann noch ein Teil des Glycerins der Kapselfüllung in die Kapselhülle und wirkt dort als Weichmacher.

Die erfindungsgemäßen Weichgelatinekapseln verlieren nach dem Trocknen praktisch kein Wasser mehr, jedoch ist es insbesondere bei Kapseln mit niedrigem Glycerinzusatz empfehlenswert, die fertigen Kapseln mit einer gas- und wasserdampfdichten Schicht zu überziehen. Diese Schicht kann beispielsweise eine luft- und gasdichte Metallfolie oder eine Kunststoffolie mit Innen- und Außenbeschichtung sein. Prinzipiell ist es auch möglich, die Oberfläche der fertigen Kapseln mit einer gas- und wasserdampfdichten Schicht zu versiegeln, die sich jedoch aufbrechen oder auflösen läßt.

Die Herstellung der erfindungsgemäßen Weichgelatinekapseln bestehend aus einer Kapselhülle und einer Kapselfüllung, die gegebenenfalls Wirkstoffe und/oder Diätmittel enthält, erfolgt dadurch, daß die Kapselfüllung aus einem Gemisch aus 2 bis 20 Gew-% Glycerin und 80 bis 98 Gew.-% Honig, Invertzucker oder Zuckersirupen besteht, in denen gegebenenfalls die Wirkstoffe und/ oder Diätmittel gelöst oder dispergiert werden und diese Gemische in an sich bekannter Weise in Weichgelatinekapseln abgefüllt werden.

Als Zuckersirupe für die Kapselfüllung kommen prinzipiell alle konzentrierten, wässrigen Zuckerlösungen, Stärkesirupe, hydrierten Stärkesirupe und Lösungen von Zuckerderivaten sowie Gemische derselben in Frage. Von besonderem Interesse sind aber vor allem Honig, Invertzucker und Gemische derselben mit Zuckersirupen.

In den nachfolgenden Beispielen sind erfindungsgemäße Weichgelatinekapseln und das Verfahren zu ihrer Herstellung näher erläutert.

## Beispiel 1

Honig oder Kunsthonig nach (DAB) werden mit 10 % Glycerin vermischt. In dieses Gemisch werden wässrige, alkoholische oder lipidhaltige Lösungen der Wirkstoffe eingerührt. Insbesondere um ein Emulgieren mit Luft zu vermeiden, kann der Mischvorgang auch mit einem Mischgerät oder Rührgerät im Vakuum erfolgen. Das fertige Füllgut wird in üblicher Weise in Weichgelatinekapseln abgefüllt. Die Zusammensetzung der Kapselhülle beträgt 45 % Gelatine, 45 % Wasser und 10 % Glycerin. Während im Gelatinesud das Verhältnis zwischen Gelatine und Glycerin 45 : 10 beträgt, ist es nach Auftrocknung der Kapseln durch Diffusion von Glycerin aus dem Kapselinhalt in die Kapselhülle auf 45 : 20 angewachsen und entspricht damit normalen Verhältnissen. Die fertigen Kapseln werden mit einer Folie aus Aluminium oder Kunststoff gas- und wasserdampfundurchlässig verpackt.

## Beispiel 2

In gleicher Weise wie in Beispiel 1 beschrieben wird Honig oder Kunsthonig mit 5, 15 und 20 % Glycerin vermischt, mit den Wirkstoffen versetzt und in Weichgelatinekapseln abgefüllt.

Die Weichgelatinekapseln mit 5 % Glycerin haben bei ungeschützter Lagerung an der Luft nur eine Haltbarkeit von einigen Wochen. Die Kapseln gemäß Beispiel 1 sowie unter Zusatz von 15 und 20 Gew.-% Glycerin sind auch ungeschützt längere Zeit haltbar, jedoch bereitet die Verkapselung des Gemisches mit 20 % Glycerin gewisse Schwierigkeiten, da offensichtlich ein zu großer Teil des Glycerins in die Kapselhülle wandert.

## P a t e n t a n s p r ü c h e

1. Weichgelatinekapseln bestehend aus einer Kapselhülle und einer Kapselfüllung, die gegebenenfalls Wirkstoffe und/oder Diätmittel enthält, dadurch gekennzeichnet, daß die Kapselfüllung besteht aus einem Gemisch aus 2 bis 20 Gew.-% Glycerin und 80 bis 98 Gew.-% Honig, Invertzucker oder Zuckersirupen, in denen gegebenenfalls Wirkstoffe und/oder Diätmittel gelöst oder dispergiert sind.

2. Weichgelatinekapseln gemäß Anspruch 1, dadurch gekennzeichnet, daß sie in gas- und wasserdampfdichten Folien eingesiegelt sind.

3. Weichgelatinekapseln enthaltend Zell- und Organbestandteile nach DE-OS 33 02 319, dadurch gekennzeichnet, daß die Zell- und Organbestandteile gelöst oder dispergiert sind in 10 bis 15 Gew.-% Glycerin und 85 bis 90 Gew.-% Honig, Kunsthonig oder Zuckerstoffen.

4. Weichgelatinekapseln gemäß Anspruch 3, dadurch gekennzeichnet, daß sie in gas- oder wasserdampfdichten Folien eingesiegelt sind.

5. Verfahren zur Herstellung von Weichgelatinekapseln bestehend aus einer Kapselhülle und einer Kapselfüllung, die gegebenenfalls Wirkstoffe und/oder Diätmittel enthält, dadurch gekennzeichnet, daß die Kapselfüllung aus einem Gemisch aus 2 bis 20 Gew.-% Glycerin und 80 bis 98 Gew.-% Honig, Invertzucker oder Zuckersirupen besteht, in denen gegebenenfalls die Wirkstoffe und/oder Diätmittel gelöst oder dispergiert werden und diese Gemische in an sich bekannter Weise in Weichgelatinekapseln abgefüllt werden.

6. Verfahren gemäß Anspruch 5, dadurch gekennzeichnet, daß die fertigen Kapseln mit einer gas- und wasserdampfdichten Schicht überzogen werden.

7. Verfahren gemäß Ansprüchen 5 und 6, dadurch gekennzeichnet, daß die Rezeptur für die Kapselhülle eine verminderte Menge Glycerin enthält.

**0199034**

Nummer der Anmeldung

EP  86 10 3010

**Europäisches Patentamt**

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| Y | US-A-2 580 683  (KREUGER)  * Insgesamt *  --- | 1,2,5, 7 | A 61 K   9/48 A 61 K   9/66 |
| Y | EP-A-0 120 248  (SCHERER)  * Seite 6, Zeile 1 - Seite 7, Ende; Seiten 13,14; Vergleichsbeispiel 1; Patentansprüche 1,2,5 *  --- | 1,2,5, 7 | |
| D,A | DE-A-3 302 319  (THEURER) * Insgesamt *  ----- | 3,4 | |

| | RECHERCHIERTE SACHGEBIETE (Int Cl 4) |
|---|---|
| | A 61 K A 23 L |

Der vorliegende Recherchenbericht wurde für alle Patentansprüche erstellt

| Recherchenort DEN HAAG | Abschlußdatum der Recherche 30-05-1986 | Prüfer BENZ K.F. |
|---|---|---|